# EUROPEAN PATENT SPECIFICATION

(11) **EP 0 691 841 B1**
(45) Date of publication and mention of the grant of the patent: **29.05.2002**
(21) Application number: 94907192.2
(22) Date of filing: 10.01.1994
(51) Int. Cl.: A61K 9/00, A61F 2/06, A61L 31/00

(54) **MEDICAMENT DISPENSING STENTS**
MEDIKAMENTE ABGEBENDE STENTS
EXTENSEURS A LIBERATION DE MEDICAMENTS

(30) Priority: 08.01.1993 US 2209; 09.09.1993 US 119407
(43) Date of publication of application: 17.01.1996
(73) Proprietor: Miravant Systems, Inc., Santa Barbara, CA 93117 (US)
(72) Inventor: NARCISO, Hugh L., Jr., Santa Barbara, CA 93109 (US)
(74) Representative: Lehmann, Klaus, Dipl.-Ing.
(86) International application number: US9400369
(87) International publication number: WO9415583

(56) References cited:
- WO-A-91/17789
- WO-A-92/11896
- WO-A-92/15342
- US-A- 4 849 226
- US-A- 4 895 724
- US-A- 5 019 075
- US-A- 5 019 090
- US-A- 5 197 977
- US-A- 5 234 456
- US-A- 5 264 214

## Description

### 1. Field of the Invention

The present invention relates to a medicament-dispensing medical implant, namely an implantable medicament dispensing stent or patch for the treatment of atherosclerosis by adjunctive photodynamic therapy generally, and more particularly to a medicament dispensing device for the prevention of restenosis following atherectomy or angioplasty.

### 2. Prior Art

Atherosclerosis is a cardiovascular disease in which deposits of plaques (atheromas) containing cholesterol, lipid material, foam cells, lipophages and proliferating smooth muscle cells are within the intima and media of large to small diameter arteries such as the aorta and the iliac, femoral, coronary and cerebral arteries. The resultant stenosis causes reduction in blood flow.

Attempts to treat atherosclerosis have included by-pass surgery wherein the diseased vascular segments are augmented by prosthetic or natural grafts. This procedure requires general anesthesia and a substantial healing period after surgery and, thus, is generally limited to cases of severe coronary artery disease.

Other approaches for the recanalization of stenotic vessels include percutaneous transluminal coronary angioplasty (PTCA), atherectomy, stenting and newer modalities of cardiovascular intervention including laser angioplasty. The word "recanalization", as used herein, means a procedure for increasing blood flow through the occluded vessel by angioplasty, including dilation or ablation or removal of occlusive material. The primary drawbacks of these methods has been restenosis. Studies have shown that restenosis, or the re-narrowing, of the internal lumen of an artery subsequent to such recanalization occurs in about 25-50% of cases where such primary treatment is performed. The result of re-stenosis is the requirement for an additional interventional or surgical procedure.

Various mechanisms can cause re-stenosis. One mechanism is rapid smooth muscle cell (SMC) proliferation at the lesion site. Smooth muscle cell proliferation is believed to occur immediately or at any time up to several hours after vessel wall injury that results from primary atherosclerotic treatment such as angioplasty. This proliferation continues for about 5-18 days depending on the individual. The cause of this rapid smooth muscle cell proliferation is believed to involve the release of various growth factors in response to the vessel wall injury. Specifically, after such vessel wall injury, some smooth muscle cells migrate to the intima where they are affected by the blood elements with which they come in contact. especially platelets and lipoproteins. Platelets contain a factor that stimulates smooth muscle cell proliferation and migration, which can result in re-stenosis.

March, et al., in U.S. Patent 5,116,864 provides a method for preventing restenosis in a patient undergoing vascular recanalization. The method comprises the systemic administration of a photoactivatable psoralen compound, preferably by an oral route, to achieve therapeutically effective serum levels. Alternatively, the psoralen may be delivered to the tissue by intra-arterial injection through a catheter. Following psoralen administration, the atheroma is illuminated with ultraviolet radiation until recanalization is complete. This is where March, et al.'s treatment ends.

Other drugs are used to prevent restenosis. One such drug is heparin. Heparin is an anticoagulant which when delivered systemically severely reduces the ability of the body to form blood clots. Devices for preventing restenosis such as implantable stents require massive doses of heparin which requires the patient to remain hospitalized for a long period of time.

Stents are presently being investigated as a treatment for cardiovascular disease. Early results indicate that stents have a similar rate of restenosis when compared to conventional interventions with the added complication of abrupt closure of the vessel. Stents have also been used as a "bail out" device to maintain the patency of a collapsing artery until another corrective medical procedure can be performed. Stents are composed of many materials including stainless steel, biodegradable polymers, collagen, gelatin, etc.

Local drug delivery devices such as suppositories and dermal patches have been used as indwelling devices. Indwelling devices to treat cardiovascular disease through delivery of local drugs are not clinically available.

One approach to the problem of sustained long term drug delivery employs implantable biodegradable polymer/drug combinations in a variety of ways to achieve a controlled regular or continuous administration of the drug. Biodegradable polymers are useful as carriers for many different types of drugs because they serve as a temporary matrix to hold the drug, but do not chemically interact with the drug. As the matrix erodes, the drugs are released and can diffuse into the tissues.

In one prior art embodiment, a synthetic (non-biogenic) biodegradable polymer matrix is homogeneously impregnated with a medicament so that the medicament is released more or less continuously and uniformly as the supporting polymer matrix erodes. In another variation of this basic idea, a single reservoir of the drug or medicament in solution is encapsulated by a semi-porous polymer matrix. The drug diffuses continuously out of the reservoir, through the polymer, and finally to the intended delivery area. Metal stents coated with bioabsorbable synthetic polymer have also been used to deliver medicament but such metal stents are optically opaque and thrombogenic. In still a further variation, tiny discrete "pockets" of the drug are encapsulated throughout the synthetic polymer. If the polymer is biodegradable then it will completely dissolve thereby releasing all of the impregnated or encapsulated drug. The above prior art devices are known in the art and are made from synthetic polymers. The problems with implants fabricated using non-biogenic material are that such prior art implants are thrombogenic and being a "foreign body" stimulate the host's inflammatory response.

A device such as a medicament-dispensing stent can also be constructed from naturally occurring biopolymers and derivatives thereof or biogenic tissue. Biological materials such as bovine and porcine tissues harvested from donor animals are commonly used for implantation into the human body. They are known to be non-thrombogenic and non-inflammatory. The porcine heart valve is one such example. Such biogenic tissues are well received and well tolerated by the host human tissues and, unlike biodegradable synthetic polymers, biogenic tissue implants are less likely to induce an inflammatory host response and are replaced over time by the host natural tissue produced *in situ.* Human tissues harvested from a human donor (autologous or heterologous) are also viable tissue types for this device.

A further prior art embodiment is known from US-Patent 5,197,977 (Hoffman, Jr. et al.) which discloses a drug delivery collagen-impregnated synthetic vascular graft. Particularly, this reference discloses a collagen-impregnated vascular graft including drug materials complexed with the collagen to be released slowly from the graft following implant. Therein, the drug materials complexed with collagen may include antithrombic agents.

Accordingly, in view of the above discussed known prior art embodiments, there is a need to address the problem of smooth muscle cell proliferation in the treatment of atherosclerosis to minimize or eliminate the occurence of restenosis.

### SUMMARY OF THE INVENTION

Photoatherolytic (PAL) Therapy is believed to be potentially effective for the prevention of restenosis. Photodynamic Therapy (PDT) has also been demonstrated to be very effective in the treatment of various cancers. In one embodiment, a stent-type device made from biogenic tissue and/or biopolymers is described which can be used to deliver a Photosensitive drug locally to a target tissue over a period of time. In another embodiment, a plug-type of implantable device is described which can be embedded in solid tissue thereafter to deliver medicament to a target. In still another embodiment the implant may take the form of a substantially planar patch.

In a particular preferred embodiment a medical implant is described for the local delivery of medicament to an intraluminal target tissue. The device and method involves the use of a bio-absorbable biogenic patch or stent which is impregnated or otherwise burdened with a photosensitizer drug, with or without complimentary medicaments, to locally deliver said drug(s) into target tissue on the vessel wall over a prolonged period of time as the stent is absorbed. Although the use of bioabsorbable, non-biological, but biocompatible stents have been proposed for such a therapy and remain a viable solution, these materials are often inflammatory to the host tissue.

Accordingly, it is an object of this invention to produce a medicament-dispensing implant which is non-thrombogenic, minimally inflammatory and generally well received and well tolerated by the human body.

It is another object of this invention to produce a medicament-dispensing implant which is absorbed by the body over time.

It is yet another object of this invention to produce a medicament-dispensing implant which can deliver medicaments substantially only to selected target tissues.

It is still another object of this invention to provide a biodegradable biogenic tissue implant which can deliver medicaments over a sustained period of time and be replaced by host tissue.

It is another object of this invention to describe a method in which this device can be used to locally deliver medicament(s) over a prolonged period of time.

It is still another object of this invention to describe a method in which this device can be used to locally deliver medicament(s) including a photosensitizer over a sustained period of time which is ultimately used in PDT.

The above referenced objectives are met by the present invention, according to which a medicament-dispensing medical implant comprises a biocompatible bioabsorbable biogenic material impregnated with at least one photosensitive medicament, wherein said biogenic material solely comprises tissue removed from a donor animal or human donor.

Accordingly, the invention describes an implantable medicament-dispensing stent, patch or plug (herein collectively "implant") for the prevention of restenosis following atherectomy or angioplasty. The implant according to the invention is comprised of bioabsorbable material containing at least one photosensitive medicament. The photosensitive medicament is selectively retained by the atheromatous smooth muscle cells and plaque. When activated by light, for example, delivered by a laser operating at a specific wavelength, the photosensitizer, which has been selectively absorbed in the atheroscleric cells, becomes toxic. In this way, the activated photosensitizer facilitates the destruction and reabsorption of the host atheromatous plaque and smooth muscle cells. The foregoing procedure is collectively termed photodynamic therapy.

In a preferred embodiment of the medicament-dispensing medical implant according to the invention the implant has a medicament-containing portion which disintegrates following implantation within a blood vessel and wherein the disintegration of said medicament-containing portion is operable for the contemporaneous release of medicament from said medicament-containing portion of said implant and wherein the medicament is a smooth muscle cell proliferation inhibitor which is substantially completely released from said medicament-containing portion within 15 days following implantation of said implant within the blood vessel.

The device of the present invention provides a means for treating vascular disease which avoids the problems and disadvantages of the prior art. The invention accomplishes this goal by providing a bioabsorbable (biodegradable) stent which releases a medicament. The medicament blocks the growth factor binding sites on the atherosclerotic smooth muscle cells (SMC) injured during the recanalization until growth factor is no longer released from the platelets in the vicinity of the injured cells. In this way, smooth muscle cell proliferation in the vicinity of the lesion site is inhibited, thereby minimizing or eliminating the occurrence of restenosis.

The device, generally comprising of a stent, is used post recanalization to: a) deliver a photosensitive medicament which blocks the chain of events linked to restenosis along with delivering other medicaments to prevent events linked to restenosis such as thrombosis and the formation of an intravascular matrix of collagen and fibrin; b) maintain the patency of the treated artery and prevent elastic recoil of the artery by adding structure and support to the vessel wall; and c) deliver and maintain a level of photosensitizer to the treatment site which inhibits smooth muscle ceil proliferation and, when activated by light energy, induces cell lysis.

In the preferred embodiment, the blocking step is accomplished by introducing a photosensitizing agent in the region of the vessel subject to the recanalization such that the agent accumulates in the atheromatous plaque and injured smooth muscle cells. The photosensitizer, accumulated in the atheromatous plaque and smooth muscle cells, blocks the smooth muscle cell growth factor binding sites to inhibit smooth muscle cell proliferation. The photosensitizer is slowly released as the drug-laden bioabsorbable wall of the vasoabsorbable stent (VAST) slowly disintegrates over a period of about 5-18 days following recanalization, which corresponds to the period needed for growth factor release from platelets to terminate and which varies among patients. The continuous readministration of photosensitizer serves to replace previously administered photosensitizer, which is cleared from the cells over time, to ensure that the growth factor binding sites are blocked until growth factor has cleared from the tissues. After the majority of the photosensitizing agent has been delivered to the surrounding smooth muscle cells from the VAST but before it clears from the atherosclerotic smooth muscle cells and plaque, the photosensitizing agent is exposed to light at a wavelength at which the photosensitizer absorbs the light causing cell lysis. Since the growth factor has cleared before atherosclerotic plaque and cell lysis, the likelihood of restenosis is significantly reduced or eliminated. The process of activating a photosensitizer with light to cause cell neurosis is called photodynamic therapy, or more particularly in the case of atherosclerosis, photoatherolytic therapy.

The advantages of the at least partially degrabable medicament dispensing stent over prior art include:
1. Bioabsorbable polymer;
2. Non-thrombogenic;
3. May be a metallic stent coated with a bioabsorbable non-synthetic polymer for reduced thrombogenicity;
4. Localized drug delivery - continuous localized time release drug delivery to affected area eliminates photosensitivity problems which arise following systemic delivery;
5. Maintain structural integrity of vessel (scaffolding);
6. Maintain patency - prevent elastic recoil;
7. Eliminate the need for serial injection of medicament;
8. Eliminate the need for serial oral administration of medicament;
9. Lower systemic doses and higher local doses of medicament;
10. Combined drug therapy (anti-proliferation, anti- recoil, anti-thrombogenicity, anti-platelet, anti-fibrin, anti-collagen); and
11. Combined drug/device therapy.

In summary, the medicament-dispensing VAST of the present invention has the potential to greatly impact the treatment of cardiovascular disease by treating the disease from a cellular cause level (the atherogensis perspective) and not merely from the conventional palliative approach.

Although the most important application of this novel method is to prevent restenosis following angioplasty or atherectomy of coronary arteries, this technique also can be applied to atherosclerotic arteries located elsewhere, such as the renal, iliac, femoral and popliteal arteries. Additionally, a vaso-absorbable stent can be used to prevent arterial occlusion after coronary by-pass surgery wherein vascular segments are replaced with prosthetic or natural grafts and growth factor is released in response to the arterial wall injury.

The above is a brief description of some deficiencies in the prior art and advantages of the present invention. Other features, advantages and embodiments of the invention will be apparent to those skilled in the art from the following description, accompanying drawings and appended claims.

### BRIEF DESCRIPTION OF THE DRAWINGS

Figure 1 depicts a stent in its non-expanded state.
Figure 2 (a,b,c) depicts an artery with an atheromatous plaque.
Figure 3 (a,b) depicts the same artery as Figure 2(a-c), but with views before and after an atherectomy procedure has been performed to removed the atheromatous plaque.
Figure 4 depicts the stent loaded over and angioplasty balloon in a non-expanded state at the lesion site post atherectomy.
Figure 5 depicts the stent expanded over the expanded angioplasty balloon at the lesion site post atherectomy.
Figure 6 depicts the stent deployed in the artery post atherectomy where it remains until it is absorbed by the artery wall thus locally delivering a cocktail of medicament continuously to the arterial wall.
Figure 7 is a partially cutaway perspective view of the stent deployed in an artery.
Figure 8 is a side view of a medicament-dispensing implant in accordance with the present invention in the form of a stent.
Figure 9 shows the stent of Figure 8 deployed on a balloon catheter.
Figure 10 is a cut-away view of a tubular tissue such as an artery housing the catheter of Figure 9 with the balloon only partially inflated.
Figure 11 is a cut-away view of a tubular tissue such as an artery housing the catheter of Figure 9 with the balloon fully inflated.
Figure 12 shows a partially cut-away view of the artery with the stent deployed in the artery adjacent to the target tissue and with the catheter removed.
Figure 13 is a partially cut-away perspective of the artery showing the stent deployed within the artery as in Figure 12.
Figure 14 is a partially cut-away perspective view of the shaft of a needle showing an implantable plug which is dimensioned to fit within the lumen of the hollow-bore needle for deployment.

### DESCRIPTION OF THE PREFERRED EMBODIMENT

### Preparation of the Biogenic Tissue

Biogenic tissue, either a portion of an exogeneous organ or endogeneous organ, such as endothelium from the innermost layer of an artery (intima), collagen, fibrin, etc. is surgically removed from a donor animal such as swine or a human donor (autologous or heterologous) and maintained in a nutrient rich solution. These viable tissues can then be "fixed" using a stabilized glutaraldehyde process at pressures less than 2mm Hg that is well known in the art and used for such implants as replacement porcine heart valves.

Alternatively, biogenic macromolecules (alternatively referred herein as "biopolymers"), which are formed by the combination of two or more naturally occurring or nonsynthetic molecules, such as collagen, chitin, chitosan or cellulose may also be used to fabricate a biogenic implant. Chitin, for example, comprises the bulk of the organic material in arthropod exoskeletons such as crab shell. If the exoskeleton is demineralized using strong acid the remaining chitinous fraction may be extracted, deacetylate (if desired), and pressed in to the desired shape for implantation. Cellulose is a polymer comprising glucose rings derived from plants. Derivatives of cellulose which may be suitable for implantation include methylcellulose, ethylcellulose, hydroxyethylcellulose, hydroxypropylcellulose and carboxymethylcellulose.

### Preparation of the Biogenic Implant

After the biogenic tissue has been prepared as described above, the biogenic tissue is soaked in a solution containing a relatively high concentration of the desired medicament(s) such as, for example, a photosensitizer (i.e. 0,1 mg of PS/ml solution - ... the desired concentration of PS in the device implantable will depend on the thickness of the lesion being treated and its location) for a period of 1-5 hours (the time will again depend on the tissue absorption characteristics and the location and application of the implant). The biogenic tissue is maintained at 37 ° C during the PS drug-burdening process while excluding light at wavelengths which might activate the PS. Other medicaments may be added to the bath permeate the implant. One such drug which is potentially complimentary to the PS is heparin which has the characteristics being an anti-coagulant, anti-platelet, anti-fibrin, anti-collagen agent and may facilitate the prevention of restenosis. New molecules having Heparin-like activity may also be employed.

### Method of Using the Biogenic Implant

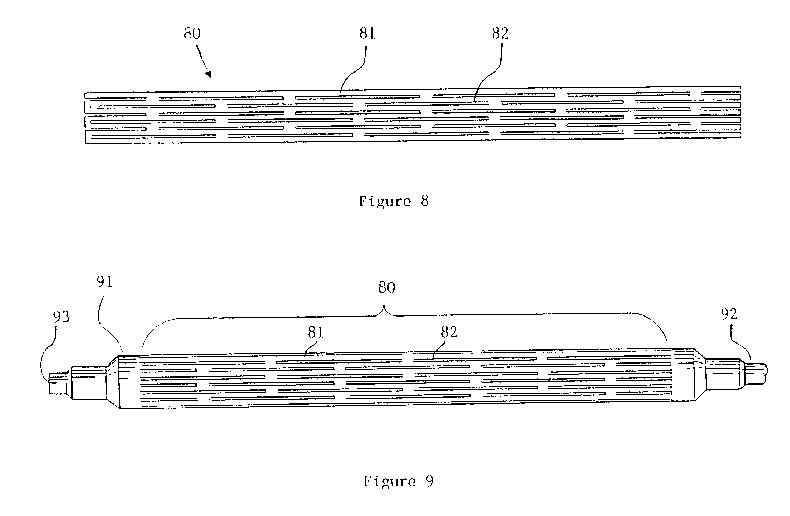

An exemplary method for using the biogenic implant according to the present invention may taught by looking first at a stent as shown in Figure 8. The stent 80 is a tubular member comprising a biogenic tissue 81 with medicament 82 incorporated therein. The medicament may be a photosensitizer which has been shown to be useful for preventing restenosis following atherectomy, or it may be any other medicament which is desirable to have released over a long period of time. The medicament may be encapsulated in discrete cells or evenly distributed throughout the body of the stent.

Turning now to Figure 9 we see the stent 80 with a balloon catheter 92 having a distal tip 93 inserted therein. The balloon catheter 92 has a balloon portion 91 which can be partially inflated so that the outer surface of the balloon portion 91 firmly and snugly engages the inner surface of the stent 80. A sheath (not shown) may or may not be used over the balloon/stent catheter to prevent the undesired deployment of the stent while advancing and positioning the catheter. Standard angioplasty procedures are used to deliver the stent through the femoral artery or other arterial point of entry and advancing the stent to the site of the target tissue.

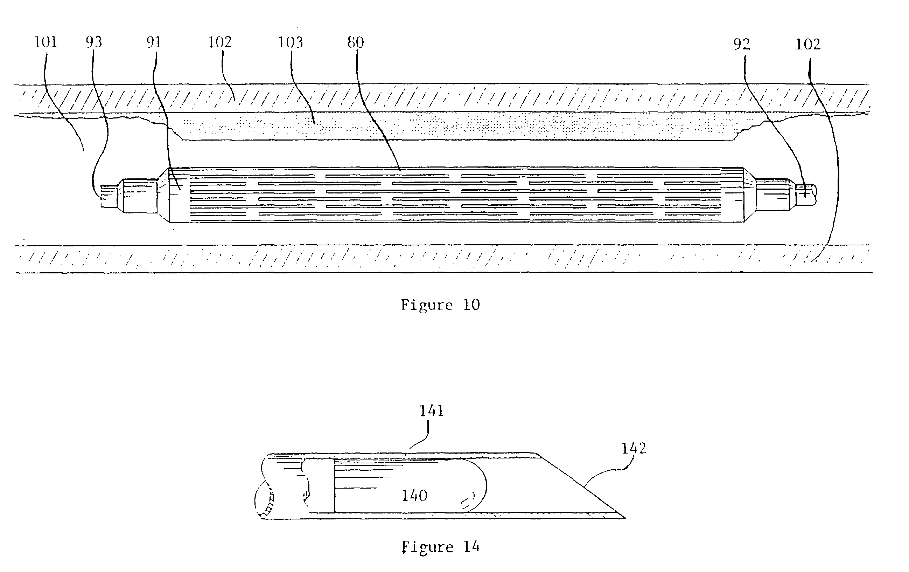

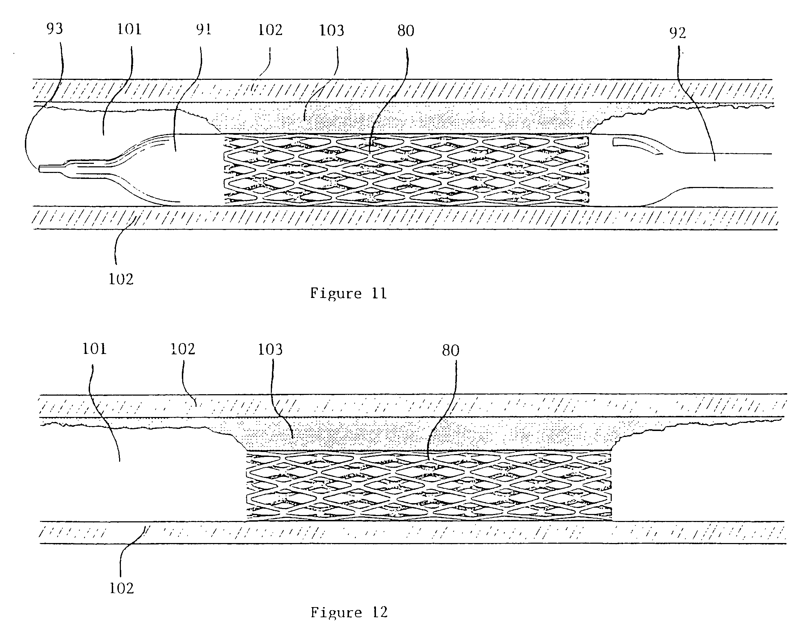

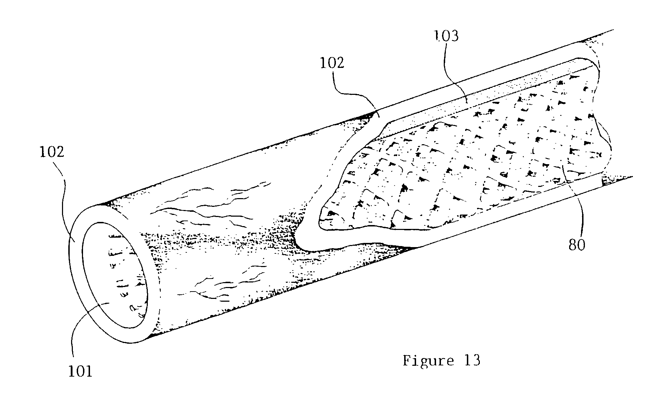

In Figure 10, a longitudinal, cross-sectional view of an arterial member is shown having an arterial wall 102 and a lumen 101. A atheromatous patch 103 on the wall 102 of the vessel (the "target tissue" in this example) has been partially removed to permit passage of the catheter 92 therethrough. The catheter 92 is advanced through the vessel until the balloon portion 91 directly underlies the area of the vessel in which it is desirable to deploy the stent 80. Once in position, the stent can be deployed by inflating the balloon member 91 so that the outer surface of the stent 80 pressed against the atheromatous lesion 101 on the wall of the vessel 102 as shown in Figure 11. The expanded stem may then be "welded" to the atheromatous tissue 103 on the wall 102 of the vessel. This "welding" may be accomplished by the application of heat to the stent. Once the stent 80 has been deployed and adhered to the target tissue 101 and the wall of the vessel, the balloon portion 91 of the catheter 92 is deflated and catheter removed as shown in Figure 12. In Figure 13 we see a fragmentary section of the vessel wall with the stent deployed therein and the balloon catheter removed. With the foregoing description in mind, I present an example of the device and method used to prevent restenosis following atherectomy.

According the present invention, photodynamic therapy is used as an adjunctive procedure to primary atherosclerotic treatment, such as percutaneous transluminal coronary angioplasty, laser angioplasty, and atherectomy to minimize or eliminate the occurrence of restenosis. Photodynamic therapy is more appropriately called photoatherolytic therapy (light induced atheromatous SMC lysis) in the specific case of cardiovascular disease.

Photodynamic therapy involves the administration of a particular medicament called a photosensitizer, usually by intravenous injection into an atherosclerotic patient. The photosensitizer, when administered intravenously, is selectively retained by the atheromatous smooth muscle cells and plaque, with little or no retention into healthy areas of the arterial wall. Generally, the photosensitizer is nontoxic to all cells when administered, but once activated by a therapeutic dose of light commonly delivered by a laser at a specific wavelength, the photosensitizer, which has been selectively absorbed in the atherosclerotic cells, becomes toxic. In this way, the activated photosensitizer facilitates the destruction and reabsorption of the host atheromatous plaque and smooth muscle cells (cell necrosis). The mechanism of cell necrosis induced by photodynamic therapy is believed to involve a photochemical reaction that produces a species of oxygen called singlet oxygen, which induces cell death.

Since the surrounding healthy tissue does not retain the photosensitizer to the extent the diseased tissue does, the therapeutic dose of light is benign to the healthy tissue regions resulting in selective necrosis. This process is disclosed using Hematoporphyrin Derivative (HPD) as the photosensitizer in U.S. Patent No. 4,512,762 to Spears, the disclosure of which is hereby incorporated herein by reference.

According to the present invention, the photosensitizer is administered in such a way as to inhibit smooth muscle cell proliferation following recanalization. It has been found that photosensitizers that have accumulated in smooth muscle cells act in the manner of a competitive inhibitor to block the growth factor binding site, thus preventing the smooth muscle cells from getting "switched on" by growth factor, which would otherwise cause rapid cell proliferation. However, since proliferation of smooth muscle cells occurs immediately or at any time up to several hours after vessel wall injury and continues for about 5 to 18 days (depending on the individual), the timing of the administration of the photosensitizer is critical to the present invention. The effect of the timing of the photosensitizer administration is discussed in detail below.

Growth factor is released in response to arterial wall injury as a result of the primary treatment (e.g., angioplasty). Since release of growth factor continues for about 5-18 days after arterial wall injury, the ubiquitous growth factors are free to "switch on" the remaining smooth muscle cells, resulting in rapid smooth muscle cell proliferation and restenosis. Thus, although concurrent or sequential recanalization with coronary angioplasty (or other interventional therapy) and photodynamic therapy (using a single administration of photosensitizer) reduces the initial proliferation of smooth muscle cells, in the long term such treatment may not be effective.

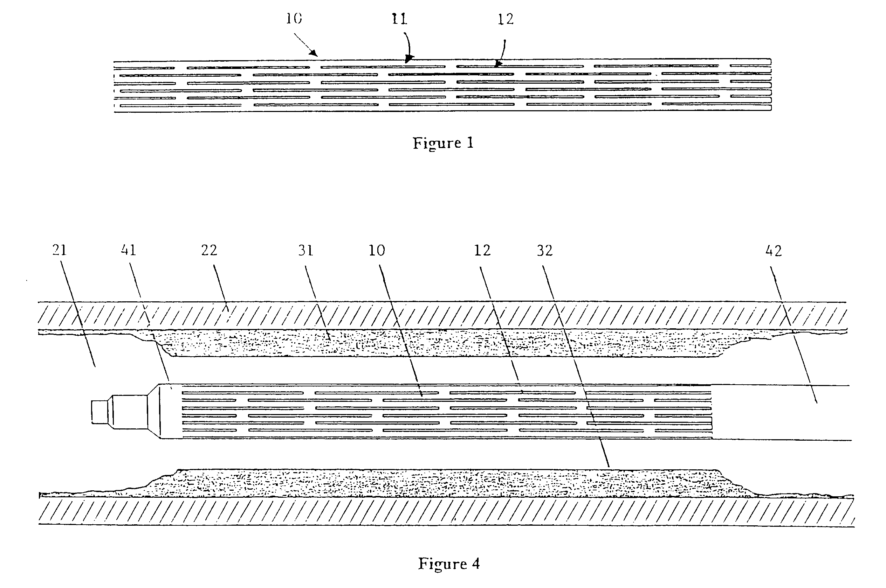

According to the present invention the preferred method of administering photosensitizer and providing adjunct therapy to cardiovascular intervention is by means of a vaso-absorbable, medicament dispensing stent. Turning now to the drawings, the "base material" of the stents 10 and 80 shown in Figure 1 and Figure 8 respectively, is preferably a polymer which has the characteristic of being absorbed by the vessel within 30-180 days. Suitable polymers or copolymers include polyglycotic/polylactic acid (PGLA), polycaprolacone (PCL), polyhydroxybutyrate valerate (PHBV) and the like. Since this therapy requires a series of pharmaceuticals to be delivered at specific times, the VAST device 10 is preferably produced in layers, each layer dispensing photosensitizer concentrations to maintain the required levels in adjacent atheromatous tissue. The stent 10 can also be made from stainless steel, titanium, NITINOL, or a variety of other metals. The drugs can be deposited on the metallic stent in a dispensing coating layer. The thickness of the drug layer 12 will depend on the time interval in which the drug is desired to be delivered (e.g. 30 days).

If the VAST 10 or 80 is made from a bio-absorbable polymer 11, the photosensitive medicament 12 will be impregnated throughout the VAST device to maintain the therapeutic levels during disintegration of the polymer. Photosensitive medicament acts as an inhibitor of smooth muscle cell (SMC) proliferation following vascular injury while also mediating SMC lysis when activated with light energy. One such photosensitive medicament is Tin Ethyl Etiopurpurin (SnET2). SnET2 has been shown to be selectively retained by atheromatous plaques. Table 1 is a list of potentially useful photosensitive medicaments.

Preferably, an anti-platelet/anti-thrombus drug such Heparin, Hirudin, tPA, Streptokinase, Urokinase, Persantine, Aspirin, etc. impregnates the VAST. Since restenosis is a response to injury, platelets are deposited at the lesion site. Platelets, along with other cellular components, release growth factors such as Platelet Derived Growth Factor (PDGF) which activates SMC proliferation. Reducing the number of platelets which get deposited at the site of injury and reducing the incidence of thrombus at the lesion site will greatly enhance the procedure. Thrombus has been associated with abrupt reclosure, embolism and the genesis of intravascular matrix which later becomes fibrous in advanced lesions.

After approximately 14 days, the SMC proliferation is replaced by the aggregation of fibrin and collagen as the dominant event in restenosis. An anti-collagen/anti-fibrin drug such as Heparin is staged to be released after about 14 days.

By taking this combined pharmacological regime, the proliferative events can be prevented while the normal healing process occurs. Once the healing has occurred and the growth factors have cleared from the lesion site, the light activation of the photosensitizer can occur if necessary.

Clinical Method: A patient presents himself/herself to the cardiac catheterization laboratory with a 90% stenosis in the mid left anterior descending (LAD) coronary artery. It is decided that the patient will be treated with an atherectomy procedure to remove a large portion of the obstruction followed by Photo-atherolytic (PAL) Therapy to prevent restenosis.

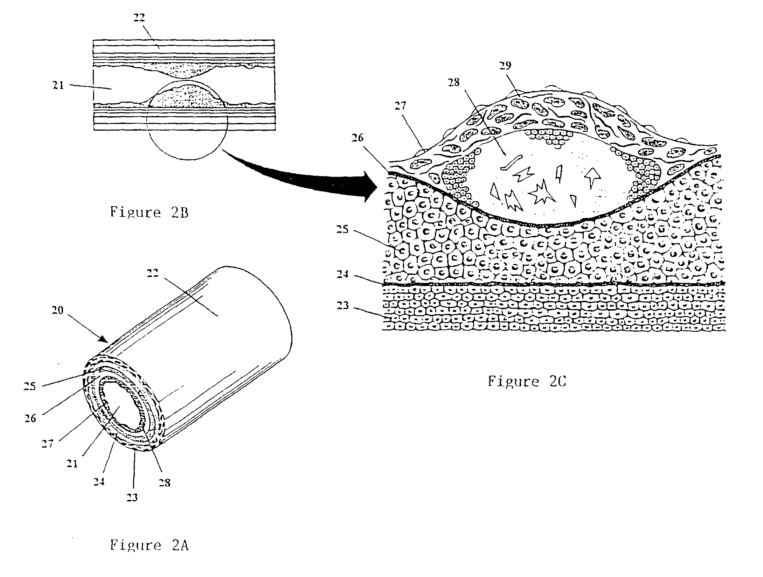

The diseased artery is shown in Figures 2a-c. The diseased artery, a segment of which is shown at 20 in Figure 2a, has an at least partially open central lumen 21 and an arterial wall 22. The wall 22 is comprised of concentric layers of tissue. The outermost layer is the adventitia 23. The external elastic lamina 24 separates the adventitia 23 from the media 25. The internal elastic lamina 26 forms the boundary between the media 25 and the intima 27. The segment of diseased vessel 20 is shown in the longitudinal cross section in Figure 2(b). The occluded portion comprising the atherosclerotic plaque is enlarged and shown in greater detail in Figure 2(c). The atheroma consists of a necrotic core 28 surrounded by a layer of smooth muscle cells 29.

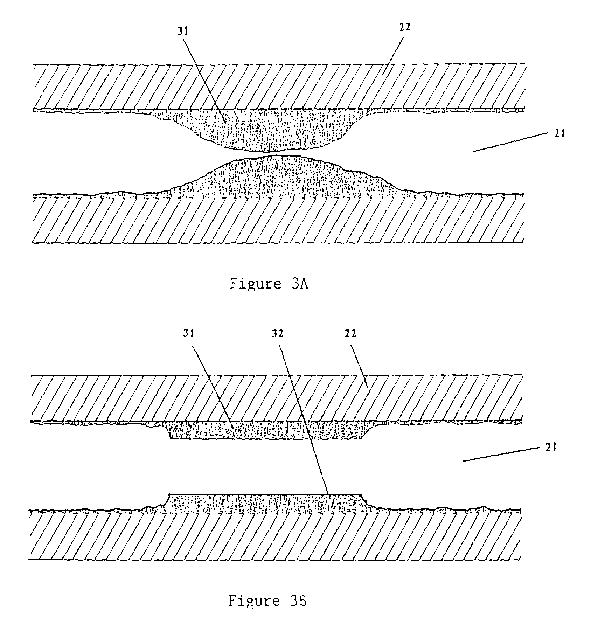

The patient is prepared per normal angioplasty procedures including the proper level of anti-coagulation prior to the atherectomy procedure. Turning now to Figure 3, the atheroma 31 is removed according to standard procedures. Following the atherectomy procedure the majority of the atheroma 31 (Figure 3(a)) has been removed as shown at 32 in Figure 3(b) leaving only a small amount of residual atheromatous tissue.

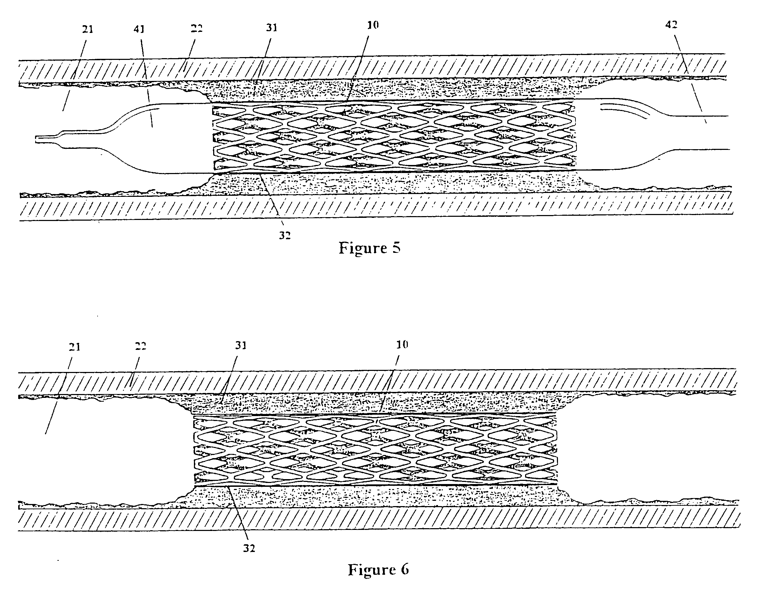

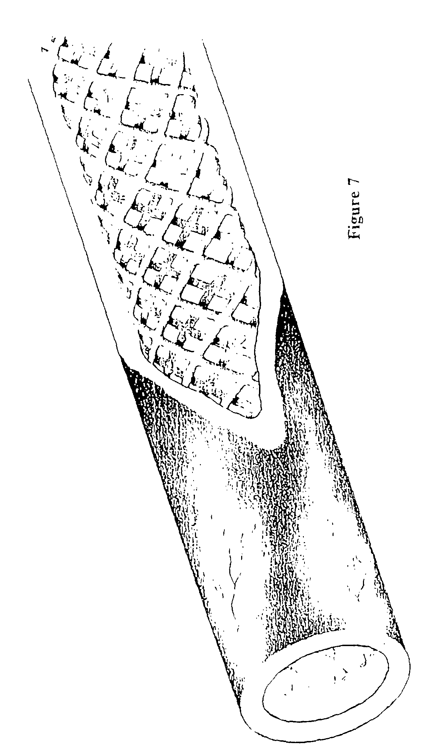

Following atherectomy as outlined above, the VAST device 10 or 80 is introduced into the vessel over a standard angioplasty balloon catheter 42 as shown in Figure 4. Once at the site of the lesion the VAST 10 is deployed by inflating the balloon portion 42 of the catheter 41 as shown in Figure 5. The balloon catheter 42 is then removed leaving the VAST in place as shown in Figures 6 and 7. The patient is then allowed to recover as with a standard atherectomy procedure. Since the VAST contains Heparin or another anti-coagulant, heavy anti-coagulation is not required as with the placement of standard metallic stents. It is advisable to prescribe an aspirin per day for the duration of the therapy. During that time the VAST releases the cocktail of therapeutic agents to the residual atheromatous lesion in a time release fashion.

Approximately 30 days following placement of the VAST, the patient presents to the cardiac cath lab. A sample of the patient's blood is taken and tested for levels of photosensitizer, anti-coagulant/anti-thrombus agent and anti-collagen/anti-fibrin agent. The level of photosensitizer (PS) is observed closely to determine if a systemic injection of the PS is required to effectively perform the Photodynamic Therapy (PDT) part of the procedure. If additional PS is required, the typical delay time for optimum photosensitization (i.e., 24 hours) must be observed.

If smooth muscle cell proliferation is controlled early enough and control maintained until growth factors clears, and if the arterial lumen is sufficiently widened, the light activation therapy may not be necessary, making this solely a pharmacokinetic therapy.

Photosensitizers which may be dispensed by the stent according to this invention include the following classes: purpurins, verdins, chlorins, phthalocyanines, phorbides, bacteriochlorophylls, porphyrins, chalcogenapyryliums, texaphyrins, xanthenes, benzophenoxasines, phenohiazines, di- and triayl-methanes, and kryptocyanines. Preferred members of the above classes are listed in the following table. The optimum light wavelength for activating each member to achieve necrosis is provided in the right column.

**TABLE 1**

| CLASS | PREFERRED COMPOUND | ACTIVATION Wavelength (nm) |
|---|---|---|
| Purpurins (metalized) | Tin Ethyl Etiopurpurin | 660 |
| | Ethyl Etiopurpurin | 695 |
| Purpurins (non-metalized) | Coproverdin-II-Tripotassium | 700 |
| Verdins | Salt | 650 |
| Chlorins | Octaethyl | |
| Phthalocyanines | Chloaluminum Sulfonated | 665 |
| | Phthyalocyanine | 660 |
| Phorbides | Mono-L-Aspoartyl Chlorin e6 | 780 |
| Bacteriochlorophylls | Bacterochlorophyll-a | 630 |
| Porphyrins | Protoporphyrin-IX | 800 |
| Chalcogenapyryliums | Chalcogenapyrylium 8b | 780 |
| Texaphyrines | Texaphyrin | 480-520 |
| Xanthenes | Rhodamine 123 | 680 |
| Benzophenoxazines | Nile Blue | 660 |
| Phenothiazines | Methylene Blue | 660 |
| Di and Triayl Methanes | Victoria Blue-BO | |
| Kryptocyanines | EDKC^{*} | 660-700 |

| | | |
|---|---|---|
| *EDKC = N, N-bis[2 ethyl-1, 3-dioxolane] kryptocyanine | | |

The preferred photosensitizing agent for incorporation in and dispensation by the VAST is Tin Ethyl Eitopurpurin having the chemical name: Ethyl 3,4,20,21-tetradehydro-4,9,14,19-tetraethyl-18,19-dihydro-3,8,13,18-tetramethyl-20-phorbine carboxylato(2-)-N²³, N²⁴, N²⁵, N²⁶ - tin(IV) dichloride. It has been found that there is little or no retention of this drug in the skin. thereby avoiding problems that can result from exposure of the patient to ordinary sunlight (i.e., activation of the photosensitizer in the skin). In addition. Tin Ethyl Etiopurpurin has a high therapeutic ratio (concentration level in diseased tissue relative to healthy tissue) as compared to other photosensitizers such as hematoporphyrin derivative (HPD). Tin Ethyl Etiopurpurin also is advantageously activated at longer wavelengths (660-690 nanometers). At these wavelengths, the light is attenuated to a much lesser degree by the blood as is the case with 630 nanometer wavelength light (the optimum wavelength for HPD, for example). As a result of using a longer activation wavelength, a substantially greater amount of light gets to the vessel wall and photosensitizer, thereby increasing procedure efficiencies.

### Example of Using the Biogenic Stent for Preventing Restenosis Following Atherectomy or Angioplasty

A drug-burdened biogenic implant in the tubular form of a stent 80 is loaded over a light diffusing catheter such as described by Narciso, Jr. in U.S. patent #5,169,395. A sheath may or may not be used over the balloon/stent catheter to prevent the undesired deployment of the stent while advancing and positioning the catheter.

To deploy the stent 80, standard angioplasty procedures are used to deliver the stent through the femoral artery or other arterial point of entry. In summary, following identification of the target tissue comprising atheromatous plaque on the wall of the vessel, the stent should be deployed in the area of injury and utilized according to the following steps:
a. the stent-deploying balloon catheter is positioned at the lesion site immediately after the completion of the angioplasty/atherectomy (the protective sheath should be pulled back to expose the PS-laden stent at this point if a sheath was used);
b. the balloon is expanded until the surface of the PS-ladened biogenic stent implant fully engages the arterial wall for the full 360 °;
c. the tissue is irradiated through the transparent balloon wall with a wavelength of light (i.e. 800-1000 nm) which produces low level heating to cause the denaturation and "welding" of the biogenic stent to the host vessel. The wavelength of the light used should not be one which activates the PS in the stent. If the activation wavelength and the welding wavelength overlap, an alternative method of heating should be employed. As example of an alternative heating method, a radio frequency (RF) heated balloon or a balloon which incorporates circulating hot fluid may be employed to effect "welding". Alternatively, a photochemical cross-linking dye may be employed to facilitate the welding process. Such dyes include, for example, brominated 1, 8-naphthalimide compounds. These dyes are activated by visible light and, following activation, covalently bind to amino acid residues, both free and in proteins, rendering them useful as protein and peptide cross-linking agents. Care should be taken to choose a photochemical cross-linking dye with an activation wavelength which will not activate the PS if one is present. The absorption maximum for the naphthalimide compounds is around 420nm, well removed from the activation wavelength of PS compounds used in PDT.
d. the balloon is then deflated and all catheters are removed from the body; and
e. (Only used for PDT applications.) A predetermined time later, the PS which has been absorbed by the vessel should be activated by means of a suitable light delivery catheter such as the catheter described by Narciso, Jr. in U.S. Patent #5,169,395 and using standard PAL Therapy techniques. The PS acts to inhibit the proliferation of cells post angioplasty thereby reducing restenosis.

The foregoing procedure for preventing restenosis is exemplary and not limiting. Similar methods can be employed to deploy a stent in non-arterial lumens such as the colon or esophagus for PDT treatment of cancer. Once deployed in the artery or other tubular tissue of the host, the stent will locally deliver the medicament(s) to the lesion area over a sustained period of time (i.e. 2 weeks to 6 months). Two weeks post stent placement, re-endothelialization should occur covering the stent with natural autologous endothelium thus encapsulating the stent in the luminal wall.

If necessary, two weeks to six months following the deployment of the stent, the patient may be brought back to the catheterization laboratory. Using standard angioplasty techniques and an invasive intravascular light diffusing catheter, the lesion site can be irradiated to receive a dose of light sufficient to activate the PS causing cell lysis and cell necrosis.

The medicament-dispensing biogenic implant of the present invention may be formed into a patch or plug for insertion into a target tissue such as a solid tumor. Such a plug 140 is shown in Figure 14. The plug 140 is dimensioned to fit within the bore of a needle 141 having a tip 142. The needle 141 may be inserted into the target tissue until the tip is embedded within or adjacent to the target tissue. The plug 140 may then be extruded through the tip 142 and the needle 141 removed.

The above is a detailed description of particular embodiments of the invention. It is recognized that departures from the disclosed embodiment may be made within the scope of the invention and that obvious modifications will occur to a person skilled in the art. The full scope of the invention is set out in the claims that follow and their equivalents. Accordingly, the claims and specification should not be construed to unduly narrow the full scope of protection to which the invention is entitled.

## Claims

1. A medicament-dispensing medical implant comprising a biocompatible bioabsorbable biogenic material impregnated with at least one photosensitive medicament, wherein said biogenic material solely comprises tissue removed from a donor animal or human donor.

2. A medicament-dispensing medical implant according to Claim 1, having a medicament-containing portion which disintegrates following implantation within a blood vessel and wherein the disintegration of said medicament-containing portion is operable for the contemporaneous release of medicament from said medicament-containing portion of said implant and wherein the medicament is a smooth muscle cell proliferation inhibitor which is substantially completely released from said medicament-containing portion within 15 days following implantation of said implant within the blood vessel.

## Patentansprüche

1. Medizinisches Implantat zur Abgabe von Medikamenten, das ein biokompatibles, biologisch absorbierbares, biogenes Material aufweist, das mit mindestens einem lichtempfindlichen Medikament imprägniert ist, wobei das biogene Material ausschließlich Gewebe umfaßt, das aus einem Spendertier oder einem menschlichen Spender entnommen wird.

2. Medizinisches Implantat zur Abgabe von Medikamenten nach Anspruch 1, das einen Medikamente enthaltenden Abschnitt hat, der sich in der Folge einer Implantation innerhalb eines Blutgefäßes auflöst und wobei die Auflösung des Medikamente enthaltenden Abschnitts für die gleichzeitige Abgabe des Medikaments aus dem Medikamente enthaltenden Abschnitt des Implantats funktionsfähig ist und wobei das Medikament ein Inhibitor für die Proliferation von Zellen glatter Muskeln ist, der innerhalb von 15 Tagen in der Folge der Implantation des Implantats innerhalb des Blutgefäßes im wesentlichen vollständig aus dem Medikamente enthaltenden Abschnitt abgegeben wird.

## Revendications

1. Implant médical à libération de médicaments comportant un matériau biocompatible, bioabsorbable, biogénique, imprégné d'au moins un médicament photosensible, dans lequel ledit matériau biogénique comporte seulement du tissu prélevé sur un donneur animal ou un donneur humain.

2. Implant médical à libération de médicaments selon la revendication 1, présentant une partie contenant le médicament, qui se désintègre à la suite de l'implantation à l'intérieur d'un vaisseau sanguin et dans lequel la désintégration de ladite partie contenant le médicament peut fonctionner pour le dégagement contemporain d'un médicament à partir de ladite partie contenant le médicament dudit implant et dans lequel le médicament est un inhibiteur de prolifération de cellules de muscle lisse, qui est substantiellement complètement dégagé à partir de ladite partie contenant le médicament dans les 15 jours suivant l'implantation dudit implant à l'intérieur du vaisseau sanguin.
